# EUROPEAN PATENT APPLICATION

(11) **EP 4 356 922 A1**
(43) Date of publication of application: **24.04.2024**
(21) Application number: 22383000.1
(22) Date of filing: 19.10.2022
(51) Int. Cl.: A61K 38/36, A61F 2/00, A61K 38/48, A61L 27/50, A61L 27/58, A61P 13/02, A61P 15/02

(54) **TREATMENT OF PELVIC ORGAN PROLAPSE**

(71) Applicant: Servicio Andaluz de Salud, 41071 Sevilla (ES); Universidad de Granada, 18071 Granada (ES)
(72) Inventor: ARRABAL MARTÍN, Miguel, 18071 Granada (ES)
(74) Representative: Hoffmann Eitle

(57) **Abstract**

**TREATMENT OF PELVIC ORGAN PROLAPSE.** The present invention refers to a method for treating pelvic organ prolapse, particularly anterior prolapse and, more particularly anterior vaginal prolapse (cystocele).

## Description

### FIELD OF THE INVENTION

The present invention refers to a method for treating pelvic organ prolapse, particularly anterior prolapse and, more particularly, anterior vaginal prolapse (cystocele).

### STATE OF THE ART

Pelvic organ prolapse refers to the situation when one or more of the organs in the pelvis (typically uterus, bowel or bladder) slip down from their normal position and bulge into the vagina.

Particularly, anterior vaginal prolapse, also known as a cystocele or a prolapsed bladder, refers to a situation when the bladder drops from its usual position in the pelvis and pushes on the wall of the vagina.

The organs of the pelvis, including the bladder, uterus and intestines, are typically held in place by the muscles and connective tissues of the pelvic floor. Anterior prolapse occurs when the pelvic floor becomes weak or if too much pressure is put on the pelvic floor. This can happen over time, during vaginal childbirth or with chronic constipation, violent coughing or heavy lifting.

Although it is a benign condition with an estimated prevalence around 5-40% of the female population, its incidence increases with age and can lead to impaired quality of life, limited physical activity and a negative impact on women's social relationships and psychological health.

Regarding its treatment, since classical surgery with stitches and anterior colporrhaphy is usually not effective, the use of non-absorbable synthetic meshes was initially introduced. However, the use of these synthetic meshes has been associated with various complications, so their use has been restricted. Following the FDA ruling, numerous scientific associations have come out in favour of the use of meshes for cystocele repair in selected cases performed by a skilled surgeon. However, many healthcare companies have withdrawn their products from the market, making synthetic meshes more difficult to make available.

So, there is nowadays a clinical need of finding alternative strategies to treat pelvic organ prolapse. The inventors of the present invention are focused on solving this problem and a new treatment strategy is herein provided.

### DESCRIPTION OF THE INVENTION

### Brief description of the invention

The present invention refers to a method for treating pelvic organ prolapse, particularly anterior prolapse and, more particularly, anterior vaginal prolapse (cystocele).

The inventors of the present invention have realized that it is necessary to reinforce the fibromuscular layer or vaginal muscularis in order to correct cystocele. To do so, the inventors of the present invention propose structurally reinforcing the muscularis layer with a composite comprising an organic matrix and the human coagulation factors fibrinogen and thrombin as active ingredients.

According to the inventors of the present invention, the composite comprising an organic matrix, and the human coagulation factors fibrinogen and thrombin as active ingredients, provides a reinforcement of vaginoplasty, due to the organic fibres and activation of clot formation over the entire subvesical surface and over the fibromuscular layer of the vagina, previously dissected.

So, the first embodiment of the present invention refers to a composite comprising an organic matrix, and the human coagulation factors fibrinogen and thrombin as active ingredients, for use in a method for the treatment of pelvic organ prolapse.

Kindly note that the composite is configured out of the body before the patient is treated.

In a preferred embodiment, the present invention refers to a composite comprising an organic matrix, and the human coagulation factors fibrinogen and thrombin as active ingredients, for use in a method for the treatment of anterior prolapse.

In a preferred embodiment, the present invention refers to a composite comprising an organic matrix, and the human coagulation factors fibrinogen and thrombin as active ingredients, for use in a method for the treatment of anterior vaginal prolapse.

In a preferred embodiment, the present invention refers to a composite, comprising an organic matrix and the human coagulation factors fibrinogen and thrombin as active ingredients, for use, according to any of the above embodiments, wherein the method comprises placing the composite in the vesicovaginal space once the vaginal layers have been dissected thus separating the fibromuscular layer from the vaginal mucosa.

In a preferred embodiment, the present invention refers to a composite comprising an organic matrix, and the human coagulation factors fibrinogen and thrombin as active ingredients, for use in a method which comprises:
a) Section the anterior wall of the vagina and dissection of the vesicovaginal space along its entire longitudinal and lateral extension up to the vaginal anchorage in the endopelvic fascia;
b) Dissection of the vaginal layers, separating the fibromuscular layer from the vaginal mucosa;
c) Place the composite in the vesicovaginal space, subvesical in its entire length and up to contact with the endopelvic fascia;
d) Plicatura muscularis layer of the vagina with continuous suture over the matrix adhered to the lower wall of the bladder, creating strong bladder suspension, tightening the vicril suture;
e) Placing a second layer of the composite over the muscularis suture of the vagina;
f) Cut the longitudinal vaginal mucosa on both sides of the initial vaginal incision;
g) Suture the vaginal mucosa over the second layer of the composite, with loose vicryl stitches;
h) Perform a vaginal tamponade for 24 hours; and
i) Removal of urethrovesical catheter.

In a preferred embodiment, the organic matrix is made of collagen.

In a preferred embodiment, the composite material further comprises the following excipients: Equine collagen, Human albumin, Riboflavine (E101), Sodium chloride, Sodium citrate (E331) and/or L-arginine-hydrochloride.

In a preferred embodiment, the organic matrix is made of collagen and the organic matrix is coated with the human coagulation factors fibrinogen and thrombin as active ingredients.

In a preferred embodiment, the organic matrix is a sponge-matrix made of collagen and the organic matrix is coated with human coagulation factors fibrinogen and thrombin as active ingredients.

In a preferred embodiment, the organic matrix is a scaffold, or a patch made of collagen and the organic matrix is coated with human coagulation factors fibrinogen and thrombin as active ingredients.

In a preferred embodiment, the composite material comprises, per cm2, 5.5 mg of human fibrinogen and 2.0 IU of human thrombin.

The second embodiment of the present invention refers to a method for treating pelvic organ prolapse, preferably anterior vaginal prolapse (cystocele by using the composite described in any of the above embodiments.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of' means including, and limited to, whatever follows the phrase "consisting of'. Thus, the phrase "consisting of' indicates that the listed elements are required or mandatory, and that no other elements may be present.
- The term "composite" refers to a material which comprises two or more constituent materials. These constituent materials are merged to create a material with properties unlike the individual elements.
- The term "matrix", in material science, is a constituent of a composite.

### Description of the figures

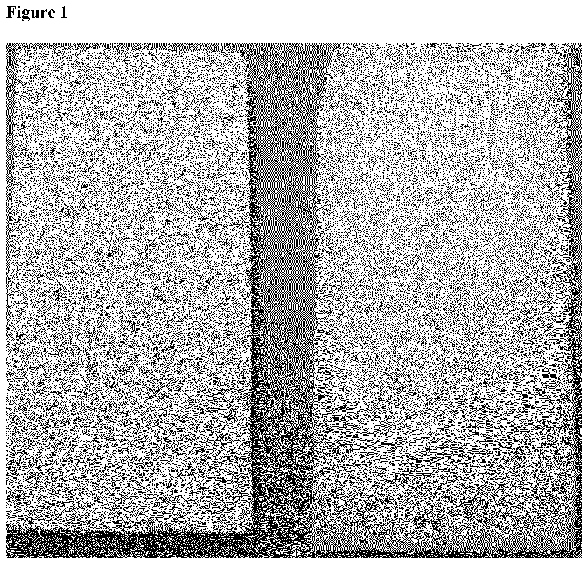

**Figure 1****.** This figure shows both parts of the composite. Left) It represents the active part of the composite. Right) It represents the organic matrix of the composite.

### Detailed description of the invention

The present invention is illustrated by means of the examples set below without the intention of limiting its scope of protection.

### Example 1. Patients

The procedure has been assayed in 6 patients. The first case was on 20 August 2020 with an average follow-up of 18 months. The six cases correspond to women aged between 50 and 64 years, with BMI between 27-35 in 4 cases and over 35 in 2 cases, diagnosed with grade II cystocele without urinary incontinence. In all cases, overactive bladder, urinary and vaginal infection were ruled out, control of eating habits was recommended to reduce BMI or at least not increase BMI and control of physical activity to avoid sudden increases in abdominal pressure, regulate bowel habits and avoid constipation.

### Example 2. Treatment

The treatment was performed under spinal anaesthesia and passed without incident in all six patients. It comprises the following steps:
a) Section the anterior wall of the vagina and dissection of the vesicovaginal space along its entire longitudinal and lateral extension up to the vaginal anchorage in the endopelvic fascia;
b) Dissection of the vaginal layers, separating the fibromuscular layer from the vaginal mucosa;
c) Place the composite in the vesicovaginal space, subvesical in its entire length and up to contact with the endopelvic fascia;
d) Plicatura muscularis layer of the vagina with continuous suture over the matrix adhered to the lower wall of the bladder, creating strong bladder suspension, tightening the vicril suture;
e) Placing a second layer of the composite over the muscularis suture of the vagina;
f) Cut the longitudinal vaginal mucosa on both sides of the initial vaginal incision;
g) Suture the vaginal mucosa over the second layer of the composite, with loose vicryl stitches;
h) Perform a vaginal tamponade for 24 hours; and
i) Removal of urethrovesical catheter.

After removal of the vaginal tamponade and urethral catheter, spontaneous urination was observed with no postvoid residue.

Vaginal rest is recommended for the first 4 weeks.

The first check-up was performed at 4 weeks, and correction of the cystocele was observed at rest and after the Valsalva manoeuvre, with no urinary incontinence after effort. After this check-up, normal physical activity was authorised without abdominal overexertion, with no limitations on social activity.

In all cases, a second check-up was performed after three months to detect possible adverse effects or complications after implantation of the collagen sponge, with good tolerance in all cases.

In all six cases the cystocele was well corrected during the follow-up period. In no case did urinary retention or voiding difficulties occur. No adverse effects occurred in any case. The collagen sponge was well tolerated, and the patients did not report dyspareunia or sexual limitation.

## Claims

1. Composite comprising an organic matrix and the human coagulation factors fibrinogen and thrombin as active ingredients, for use in a method for the treatment of pelvic organ prolapse.

2. Composite comprising an organic matrix and the human coagulation factors fibrinogen and thrombin as active ingredients for use, according to claim 1, in a method for the treatment of anterior prolapse.

3. Composite comprising an organic matrix and the human coagulation factors fibrinogen and thrombin as active ingredients for use, according to any of the previous claims, in a method for the treatment of anterior vaginal prolapse.

4. Composite comprising an organic matrix and the human coagulation factors fibrinogen and thrombin as active ingredients for use, according to any of the previous claims, wherein the method comprises placing the composite in the vesicovaginal space once the vaginal layers have been dissected thus separating the fibromuscular layer from the vaginal mucosa.

5. Composite comprising an organic matrix and the human coagulation factors fibrinogen and thrombin as active ingredients for use, according to any of the previous claims, wherein the method comprises:
a) Section the anterior wall of the vagina and dissection of the vesicovaginal space along its entire longitudinal and lateral extension up to the vaginal anchorage in the endopelvic fascia;
b) Dissection of the vaginal layers, separating the fibromuscular layer from the vaginal mucosa;
c) Place the composite in the vesicovaginal space, subvesical in its entire length and up to contact with the endopelvic fascia;
d) Plicatura muscularis layer of the vagina with continuous suture over the matrix adhered to the lower wall of the bladder, creating strong bladder suspension, tightening the vicril suture;
e) Placing a second layer of the composite over the muscularis suture of the vagina;
f) Cut the longitudinal vaginal mucosa on both sides of the initial vaginal incision;
g) Suture the vaginal mucosa over the second layer of the composite, with loose vicryl stitches;
h) Perform a vaginal tamponade for 24 hours; and
i) Removal of urethrovesical catheter.

6. Composite comprising an organic matrix and the human coagulation factors fibrinogen and thrombin as active ingredients for use, according to any of the previous claims, **characterized in that** the organic matrix is made of collagen.

7. Composite comprising an organic matrix and the human coagulation factors fibrinogen and thrombin as active ingredients for use, according to any of the previous claims, wherein the composite material further comprises the following excipients: Equine collagen, Human albumin, Riboflavine (E101), Sodium chloride, Sodium citrate (E331) and/or L-arginine-hydrochloride.

8. Composite comprising an organic matrix and the human coagulation factors fibrinogen and thrombin as active ingredients for use, according to any of the previous claims, **characterized in that** the organic matrix is made of collagen and the organic matrix is coated with the human coagulation factors fibrinogen and thrombin as active ingredients.

9. Composite comprising an organic matrix and the human coagulation factors fibrinogen and thrombin as active ingredients for use, according to any of the previous claims, **characterized in that** the organic matrix is a sponge-matrix made of collagen and the organic matrix is coated with human coagulation factors fibrinogen and thrombin as active ingredients.

10. Composite comprising an organic matrix and the human coagulation factors fibrinogen and thrombin as active ingredients for use, according to any of the previous claims, **characterized in that** the organic matrix is a scaffold or a patch made of collagen and the organic matrix is coated with human coagulation factors fibrinogen and thrombin as active ingredients.

11. Composite comprising an organic matrix and the human coagulation factors fibrinogen and thrombin as active ingredients for use, according to any of the previous claims, wherein the composite material comprises, per cm², 5.5 mg of human fibrinogen and 2.0 IU of Human thrombin.
